# EUROPEAN PATENT SPECIFICATION

(11) **EP 4 400 067 B1**
(45) Date of publication and mention of the grant of the patent: **08.07.2026**
(21) Application number: 24150248.3
(22) Date of filing: 03.01.2024
(51) Int. Cl.: A61B 18/20

(54) **SKIN TREATMENT DEVICE**
HAUTBEHANDLUNGSVORRICHTUNG
DISPOSITIF DE TRAITEMENT DE LA PEAU

(30) Priority: 12.01.2023 GB 202300486
(43) Date of publication of application: 17.07.2024
(73) Proprietor: iPulse Limited, Swansea, SA1 8QB (GB)
(72) Inventor: JONES, Stuart Terry, Swansea, SA1 8QB (GB); DAVIES, Paul Edward, Swansea, SA1 8QB (GB)
(74) Representative: Murgitroyd & Company

(56) References cited:
- EP-A1- 2 332 613
- WO-A1-2008/146789
- GB-A- 2 604 876
- US-A1- 2015 360 058

## Description

The present invention relates to a skin treatment device, preferably a skin treatment device for treating unwanted hair, and preferably comprising an Intense Pulsed Light (IPL) device.

Skin treatment devices are known in the art for treatment of, for example, cosmetic applications such as hair depilation, minimisation of skin blemishes or skin rejuvenation, as well as dermatological treatment of skin conditions such as acne or rosacea. The skin is exposed to dosages of radiation from a light source such as a flashlamp or laser where the radiation is targeted to the skin and the energy intensity and pulse duration is controlled. In hair depilation, the radiation source is targeted to cause heating of the hair root causing the hair root to die.

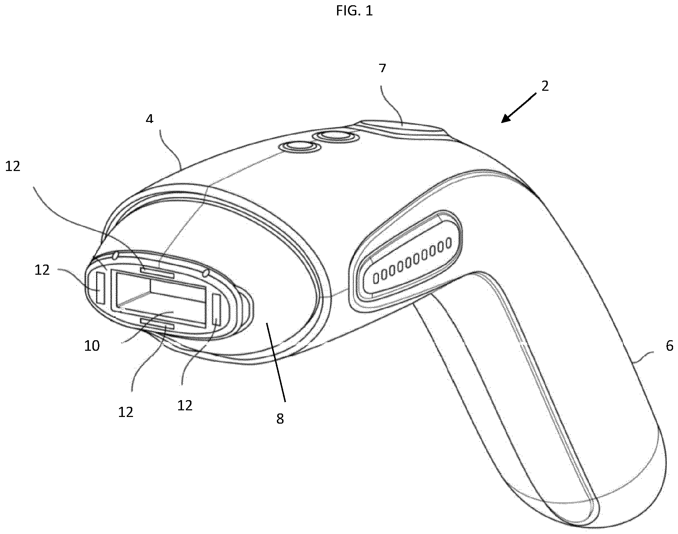

Safety of skin treatment devices is paramount and is particularly important for devices designed for home use. Home use devices require 'good skin contact' before allowing the device to emit radiation to the skin. Good contact can be defined as a condition where the light output area is sufficiently covered by skin that any stray optical radiation is below harmful levels. As such, safety features are implemented so that the device will not emit radiation unless the device is in contact with a user's skin to minimise stray optical radiation from the device in operation which may be at unsafe levels for the eyes. This is typically achieved through the provision of multiple sensors adjacent each side of the output window (for example above, below and to either side of a rectangular output window) in the head of the device where a surface must be detected by each sensor as a requirement for radiation to be emitted. If one sensor does not measure a threshold value, then it is determined by the control system of the device that there is no skin contact and firing is prevented. This is to prevent the device firing when good contact with the skin is not achieved with the associated risk of the emission of potentially harmful levels of stray optical radiation. Stray optical radiation can be defined as light emitted by the device that is intended to be absorbed by the target skin area for treatment purposes, but either misses the intended target, is reflected or remitted from the target. Various sensors may be utilised such as capacitive contact sensors or proximity sensors. Capacitive contact sensors are often utilised, and Figure 1 is a schematic illustration showing a skin treatment device 2 comprising a housing 4 shaped to be grasped by a user by a handle 6 having a forward end 8 comprising an output window 10 for emission of light energy pulses onto the skin. Positioned around the output window in the forward end 6 of the housing 4 are multiple sensors 12 capable of detecting skin contact. Assuming a threshold capacitance is measured from the sensors, then the control system in the device does not prevent emission of an energy pulse as it is deemed there is contact with the skin and emission of an energy pulse is safe.

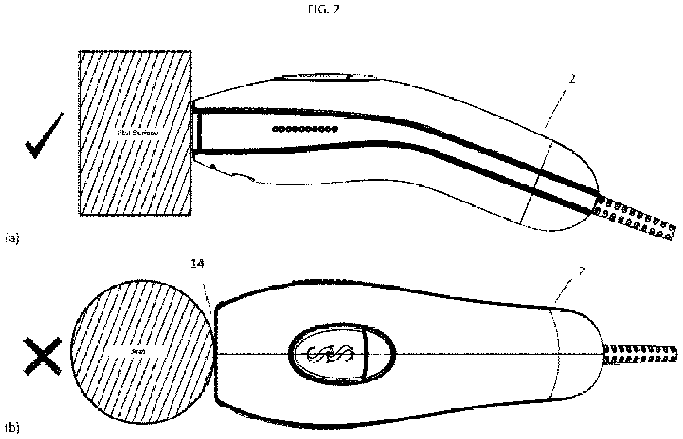

Whilst the safety features of the device limit stray radiation, there is an impact on useability. For body areas with a large flat surface, usability is good as a user can readily position the device such that all of the sensors contact the body thus allowing emission of radiation and ease of treatment. However, on more angular or bony areas of the body it is more difficult to orient the head so that all the sensors are in body contact and emission of radiation is prevented. This causes frustration for the user, and the efficacy of the treatment is reduced as difficult to treat body areas are missed. Overall usability is therefore decreased. This is clearly shown in Figure 2a where the device can readily treat a relatively flat area such as a user's calf. This compares with Figure 2b which shows attempted treatment of an arm where the gap 14 between the shin and sensors means the control system prevents emission of a light energy pulse.

Existing solutions have been proposed. For example, adaptors have been utilised that selectively secure to the front end of a device and have a curved leading edge thereby providing better communication with a curved surface of a user. This type of arrangement works for specific body geometries however is limited in suitability meaning multiple different adaptors are required leading to a significant lack of useability. Alternatively, a head portion of a device can in some circumstances be removed and replaced with a head portion having a smaller output transmission window and skin contact surface thereby enabling more complex body geometries to be treated as a result of the smaller contact area. This results in increased treatment time for the areas treated thereby reducing usability.

WO2008146789 describes a light epilation apparatus that performs epilation treatment by irradiating light. The light epilation device has a light shielding portion 6 movably supported by the device main body 1. The light shielding part 6 is formed in a tubular shape with a flexible elastic body such as rubber or elastomer and the light shielding part 6 is arranged so as to surround the light irradiation port 3 on the outer circumferential surface of the device main body 1.

Aspects of the present invention address the problems identified above or at least provide a useful alternative.

According to a first aspect of the present invention there is a skin treatment device for delivery of light energy pulses to a subject's skin as defined in claim 1.

It will be appreciated that the or each skin contact sensor may carry one or more individual sensors.

The skin contact surface preferably faces outwardly from the projection.

The provision of the skin contact portion that carries one or more sensors being at least partially moveable means that the projections can dynamically move to adapt to the geometry of the skin. Undulations in the shape of the body are therefore accommodated and can be treated safely and with ease of operation. This therefore increases usability and at the same time reduces the possibility of stray light escaping. As the projection deflects, the output window can move into a closer proximity to the skin.

The or each projection beneficially acts as a light guide for guiding the light energy pulses to the skin. Correspondingly, the or each projection also acts to prevent escape of stray light that has been output from the output window.

It will be appreciated that as the projection moves the carried sensor(s) also moves. The at least one sensor is preferably embedded in the skin contact surface. The sensor is beneficially retained by the projection, and is further beneficially held in a fixed position relative to the skin contact surface. The device comprises at least a first and a second projection, where the first and second projection deflect independently of one another.

It will be appreciated that the at least one sensor is carried by a portion of the projection that deflects.

It will be appreciated that the projections projects forwardly from the housing. This forward projection is in a direction in which light is emitted from the transmission window. The projections acts to guide the light emitted from the output window. The skin contact portion extends forwardly in a direction at least partially in a direction perpendicular to a plane of the transmission window. The projections may extend around the entirety of the output window.

The skin treatment device preferably comprises a plurality of sensors. The plurality of sensors may comprise proximity sensors (such as capacitive sensors) and/or optical proximity sensors depending on the specific functionality required. However, in any event it is beneficial that the sensors can be used to determine proximity of a surface (skin) to the sensor. If an optical proximity sensor is utilised, additional functionality may be provided such as the ability to use the sensor output (reflectance) in determination of the skin tone and therefore control the energy output by the light source dependent upon the skin tone. The sensor(s) may be a proximity or contact sensor for detecting the presence of skin. An example of a typical sensor utilised in the present device is a capacitive proximity sensor.

The one or more sensors preferably comprises at least first and second sensors carried by the at least one projection on diametrically opposing first and second sides of the output window.

The at least one projection preferably comprises at least a first and second projection independently moveable relative to each other. It will be understood that by providing first and second projections the provision of a third or more projection is not excluded. However, for skin treatment device that is portable and handheld, limitation to two projections only have been found to be beneficial. The first and second projection are each preferably positioned on opposing sides of the output window. The output window preferably comprises a length dimension and a width dimension, where the length dimension is greater than the width dimension, and the length dimension extends between the first and second projections. The first and second projection may comprise first and second jaws. A recessed portion is preferably defined between the first and second portions.

The or each projection is preferably arranged to be received into a corresponding opening in the housing, where the projection in the rest position extends outwardly from the opening, and in the deflected position the projection is at least partially withdrawn into the opening.

The at least one projection is preferably rigid. This means that the at least one projection itself is not deformable. The at least one projection is beneficially biased to the rest position by a biasing arrangement. The biasing arrangement preferably comprises a spring.

The at least one projection is preferably pivotally mounted to the housing. The skin contact surface preferably comprises a leading edge and a trailing edge, where the leading edge projects further outwardly from the housing in the rest position. The trailing edge is beneficially closer to the output window than the leading edge. The leading edge (and/or trailing edge) may be generally parallel to an edge of the output window.

The housing has a forward contact surface for contacting a user's skin, where the output window is defined in the forward contact surface. The forward contact surface is at least partially convex. The output window preferably has a solid window therein for allowing emission of light from the housing, and the solid window may also be contoured to match the geometry of the forward contact surface.

The at least one projection may be elastically deformable and conform to the geometry of a user's skin. The at least one projection may comprise an elastomeric material such as rubber. The at least one projection may extend around the entire output window. The at least one projection may comprise a single integrated projection.

Controlling operation of the device may comprise one or both of determining whether or not the flashlamp can emit a light energy pulse and determining properties of that pulse (e.g. fluence).

The at least one projection is preferably non-detachable from the housing under normal operational use.

The device is preferably an Intense Pulsed Light (IPL) device.

Aspects of the present invention will now be described by way of example only with reference to the accompanying figures where:
Figures 1 presents a skin treatment apparatus describing the general functionality of a known skin treatment apparatus.
Figures 2a and b demonstrate the ease of treatment of a relatively flat skin area compared to curved area of the skin for a prior art device.
Referring to Figure 3a and b there is perspective view of a device and a forward end of a device according to an illustrative embodiment of the present invention.
Figures 4a-b show the mechanism by which the projections can deflect relative to the body of the device, where the body of the device has been removed for clarity purposes.
Figures 5a-c show an embodiment of the present invention is presented in side view, perspective view and cross sectional view respectively. Each view shows the device in three different operational states depending on the curvature of the body.
Figure 6 is a further illustrative embodiment of the present invention.
Figure 7a and b is a schematic representation of an illustrative embodiment of the present invention in a rest and deflected configuration respectively.
Figure 8a and b is a schematic representation of an illustrative embodiment of the present invention.

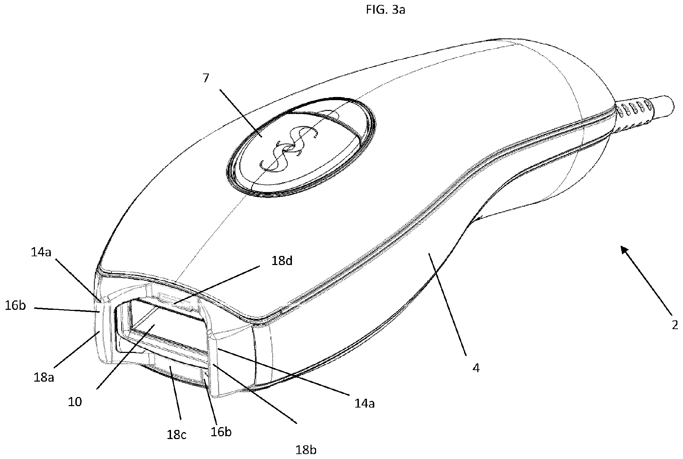

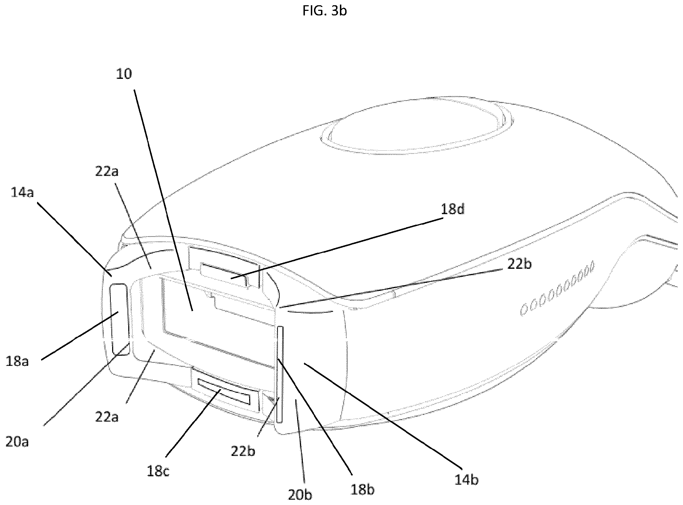

Referring to Figure 3a and b schematically presented is the entire device and forward end respectively of a skin treatment apparatus that may be used for treating skin disorders and conditions, and even more beneficially is suitable for cosmetic purposes such as hair depilation. The device 2 comprises a housing 4 and a light source (not shown) accommodated by the housing 4 such as a discharge lamp or flashlamp. The flashlamp is arranged to generate high intensity pulses of optical radiation. The housing 4 comprises a handle 6 meaning that the housing 4 can be manipulated to be positioned appropriately on a user and is particularly suitable for the home market as the device is handheld, relatively small and portable. A light output window 10 or transmission window 10 allows the passage of high intensity pulses of optical radiation therethrough, where the window 10 typically measuring 30mm in width and 10mm in height. The cross-sectional area of the light output aperture/transmission/output window 10 is effectively the treatment area. The light emitting element is housed by the housing 4 together with the control system that controls discharge of the light emitting element.

The head portion of the device 2 in one illustrative embodiment comprises a first projection 14a and second projection 14b arranged on opposing sides of the output window 10, with each protrusion providing a skin contact surface 16a, 16b. In use the skin contact surface bears against the user's skin. The first and second projections 14a, 14b define opposing jaws between which the body portion of the user is received. Within the projections 14a, 14b are first and second sensors 18a, 18b which for providing associated sensing zones. The sensors are embedded into the projections and form part of the skin contact surface of the projections. Additionally, third and fourth sensors 18c, 18d are provided on opposing sides of the output window 10 (which surface also bears against a user's skin in operation) within the forward end of the housing 4 itself. The sensors may take different forms dependent upon the device in which it is utilised. For example, the sensors may simply comprise multiple proximity sensors in the form of capitative proximity/contact sensors each having a sensing zone where the control system requires a predetermined capacitance to be measured from each sensing zone which is indicative of contact with a user's skin. Accordingly, the sensors may be embedded into the surface, project from the surface or sit behind the surface. In the illustrative embodiment these sensors are 18a-18c and are embedded. Assuming a threshold value is measured, then the control system enables firing of the flashlamp to emit a light energy pulse. However, one or more alternative or additional skin parameters may be sensed, and in the illustrative embodiment this is sensor 18d. For example, this sensor 18d may comprise an optical reflectance sensor often referred to as a skin tone sensor or sometimes again a proximity sensor and can be used in the alternative to or in tandem with one or more other sensor types such as capacitive sensors. In the embodiment presented, there are three capacitive proximity sensors and one optical proximity sensor (or 'skin tone sensor' 18d). A skin tone sensor includes a transmitter arranged to transmit sensing radiation through the sensor window onto the skin to be treated. The sensor 18d further includes a receiver such as a photodiode arranged to receive radiation reflected from a skin surface. Intensity of the received radiation is found to be representative of the tone of the skin, for example a light skin tone will reflect more than a dark skin tone. The intensity of the received radiation can be processed by the control system using a processor provided thereby and compares the intensity with a calibrated set of intensity measurements to determine a sensed skin tone, which is then stored in a memory of the control system. The treatment light pulse energy then outputted to the skin can be controlled and is thus dependent on the sensed skin tone thus ensuring optimised treatment for the specific skin tone to be treated.

The projections 14a, 14b each deflect between the rest and deflected position independently of one another. The projections 14a, 14b are each positioned on opposing sides of the output window 10. The output window 10 comprises a length dimension and a width dimension where the length dimension is greater than the width dimension. The length dimension is defined between opposing spaced apart ends and the width dimension is also defined between opposing spaced apart ends. The first and second projections 14a, 14b are positioned so that they are outwardly of the opposing ends. The first and second projection 14a, 14b effectively form first and second jaws, and a recess is defined between the first and second portions. Each of the projections 14a, 14b extend outwardly from the housing 4 and provide a skin contact surface 20a, 20b at a furthermost projection. The shape of the projections tapers inwardly towards the housing 4 and toward the other projection lengthwise parallel to the opposing ends of the width dimensions. This tapered portion 22a, 22b also provides a skin contact surface, and by providing such a shape the possibility of light escaping during operation is minimised. The skin contact surface 20a, 20b may have a tactile surface finish to enable sliding over the skin. Such a surface finish may comprise silicon for example.

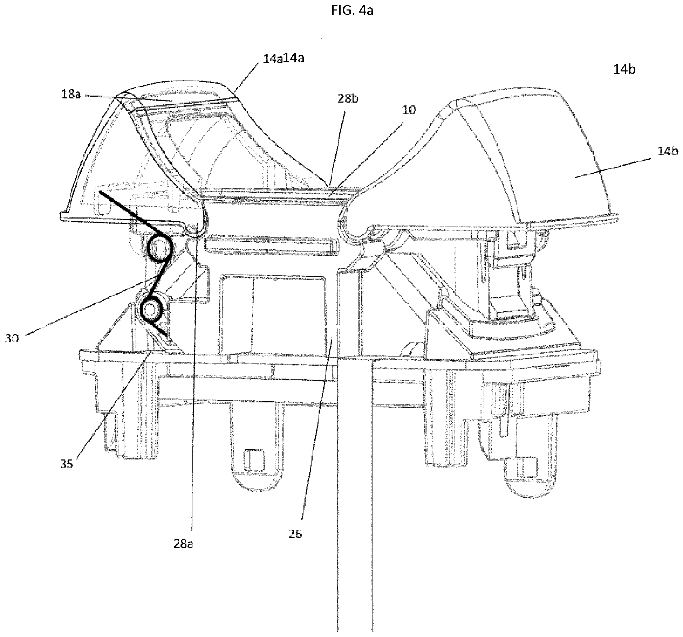

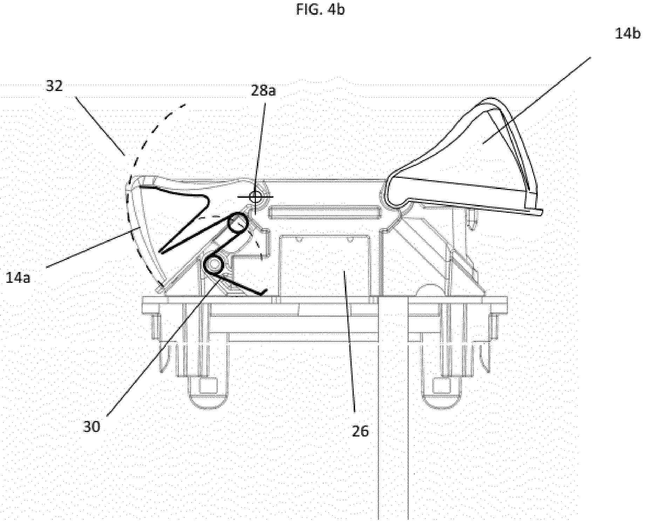

Referring now to Figures 4a-b, the mechanism by which the projections can deflect relative to the body of the device according to an illustrative embodiment is demonstrated, where Figure 4a shows the projections in the rest or fully extended configuration, and Figure 4b demonstrates one of the projections in the fully depressed configuration. It will be appreciated that most of the device has been removed for clarity purposes. Figure 4a shows both projections 14a, 14b in the rest/extended configuration, and projection 14a is shown in a see-through manner so that all components can be readily identified. It will be understood that both projections 14a, 14b are received into corresponding recesses in the housing 4 in the deflected position, which recesses are not shown in Figure 4.

The projection 14a is pivotally mounted to frame 26 at first and second pivot points 28a, 28b located at opposing sides of the width dimension of the output window 10. A biasing element 30 in the form of a double coil spring is provided for biasing the projection 14a toward the rest/extended configuration as shown in Figure 4a. Referring to Figure 4b, the spring is biased toward the contracted configuration by action of a force on the contact surface 20a and the projection 14a has therefore arced as shown by dashed line 32 into a fully deflected configuration. Upon removal of the applied force, the spring 30 returns the projection 14a to the extended configuration. User body portions that do not bias the projections to the fully deflected state are accommodated for by the spring 30 which provides a proportional reaction force to the associated projection ensuring the skin contact surface 20a,20b aligns as closely as possible to the user body portion.

Further, the embodiment presented in figure 4 has the optional benefit of allowing transmission of a sensor output through the described functional components. For example, in one embodiment the output of the sensor 18a can be transmitted through the spring to a receiving circuit 35 that interfaces with the control system.

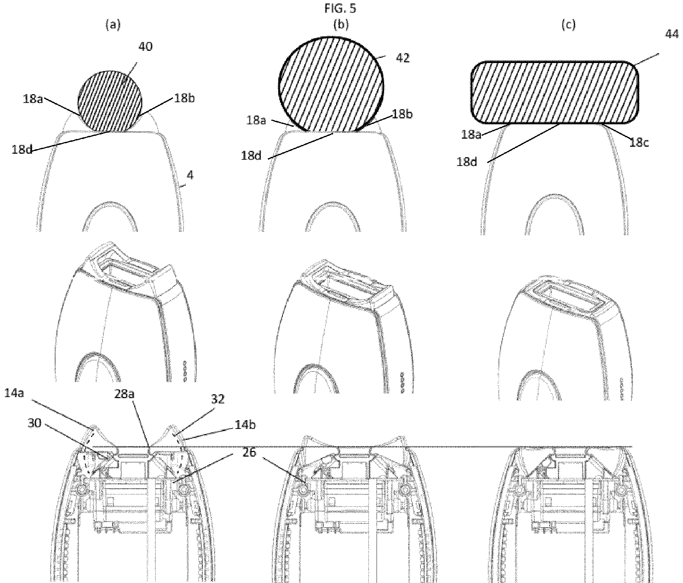

Referring now to Figures 5a-c, an embodiment of the present invention is presented in three operational states respectively, where a side view, perspective view and cross-sectional view is shown. In Figure 5a, a highly curved surface is contacted that may for example by a user's shin bone 40. The approximate diameter of the body portion may be around 40mm. When communicating with such a body part, the projections 14a, 14b are fully extended and are in a rest configuration and where operation is the device is enabled as all sensors 18a-d are in close communication with the body portion. Furthermore, stray light emitted from the output window will be minimised. Referring to Figure 5b, the body part 42 has a medium curvature of approximately 100mm diameter and may be a different part of a user's leg. The projections 14a,14b are partially deflected again meaning that good body contact is ensured, and the device can emit an energy pulse to the skin. Referring to Figure 5c, the body portion 44 is completely flat and as such the projections 14a, 14b are fully deflected and a generally planar contact surface is presented onto the skin of the user.

It will be appreciated that in alternative embodiments an alternative number of projections may be disposed around the output window. As an example, additional projections may be disposed above and below the output window transversely (i.e. across the width).

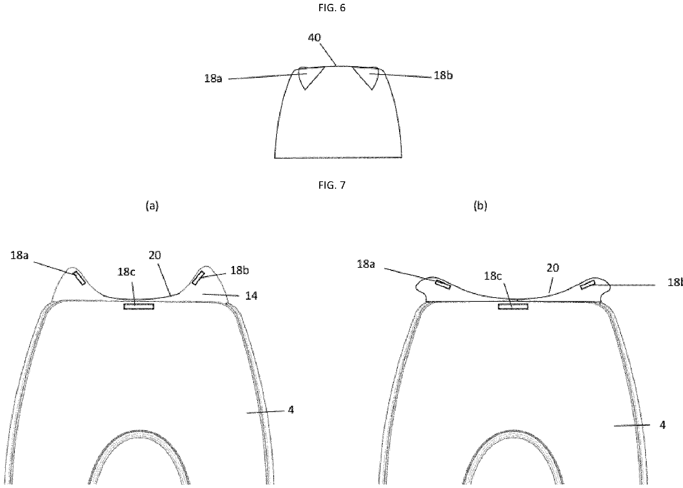

It will be appreciated that there are other body portions with geometries that lie outside of the extremes presented in Figure 5. In alterative embodiments the extremes of movement would be re-defined to accommodate such body portions. For example, referring to figure 6, in an illustrative embodiment the user contact surface of a forward end 40 of the housing may be curved, with this curvature being convex in nature to accommodate convex user body portions, such as the inside of the knee. The projections 14a,14b are shown in the deflected configuration.

In an alternative embodiment of the skin treatment device the one or more projection has a flexible instead of rigid geometry. For example, referring to figure 7, a single flexible projection is provided and carries the sensors 18a-d. In this embodiment the entirety of the projection 14 deflects from the rest configuration shown in Figure 7a to the deflected configuration as shown in Figure 7b. User body portions that do not conform to the "neutral" or "maximum" states of engagement are accommodated for by flexure of the projection 14, ensuring the skin contact surface 20 conforms to the user body portion. The flexible protrusion in a such an embodiment could be manufactured from an elastomeric material and mechanically or chemically interlocked with the housing 4.

In another embodiment, there are a plurality of flexible projections like the embodiment as presented in Figures 3-6 utilising a pair of opposing flexible projections 14a, 14b on opposing sides of the output window 10 capable of independently deflecting to conform to the body geometry of a user.

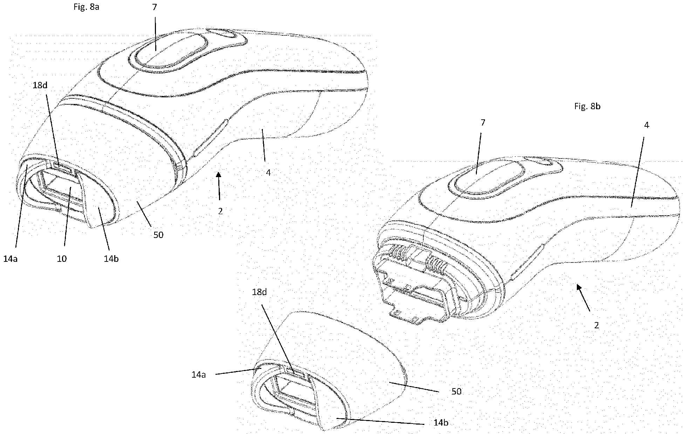

Referring to Figure 8a and b, another embodiment is presented whereby projections 14a and 14b are carried by a head 50 configured to releasably couple to the housing 4. Figure 8a shows the head 50 coupled to the housing 4, and Figure 8b shows the head decoupled from the housing 4. In a coupled configuration the device is operable, and in a decoupled configuration the device is configured to be inoperable. There are various benefits associated with such an arrangement. For example, multiple heads may be provided whereby different heads have different size output windows 10 enabling use of the appropriate head for treatment of a particular body area, where a smaller output window 10 is suitable for more awkward body areas such as upper lip. A replacement head may or may not require the provision of the projections 14a,b. Alternatively a replacement head could have a different version of the projections 14a,b such as those described with respect to Figure 7.

Aspects of the present invention have been described by way of example only and it will be appreciated to the skilled addressee that modifications and variations may be made without departing from the scope of protection afforded by the appended claims.

## Claims

1. A skin treatment device (2) for delivery of light energy pulses to a subject's skin, the skin treatment device comprising:
- a housing (4);
- a light source housed within the housing for discharging light energy pulses;
- a control system for controlling discharge of the light source to deliver the light energy pulses;
the housing (4) having an output window (10) for transmission of the light energy pulses emitted by the light source to external of the housing onto the subject's skin, the skin treatment device further comprising at least one projection (14) projecting outwardly from the housing relative to the output window (10) and the or each projection having a skin contact surface (16), wherein the or each projection is at least partially moveable from a rest position to a deflected position such that the projection deflects to accommodate the geometry of the subject's skin, the projection (16) further carrying a sensor (18) and where the control system is arranged to receive one or more sensor outputs from the sensor and based on the one or more sensor outputs control operation of the skin treatment device, **characterised in that** the one or more projection comprise at least a first and a second projection (14a, 14b), where the first and second projection deflect independently of one another.

2. A skin treatment apparatus according to claim 1 wherein each of the skin contact surfaces (16a, 16b) faces outwardly from the respective projection (14a, 14b).

3. A skin treatment apparatus according to claim 1 wherein the sensor (18) is retained by the projection (14) and is further held in a fixed position relative to the skin contact surface (16).

4. A skin treatment apparatus according to any preceding claim wherein the at least first and second projections (14a, 14b) extend around the entirety of the output window (10).

5. A skin treatment apparatus according to any preceding claim wherein the one or more sensors (18) comprises at least first and second sensors (18a, 18b) carried by the first and second projection (14a, 14b) on diametrically opposing first and second sides of the output window (10).

6. A skin treatment apparatus according to any preceding claim wherein the output window (10) comprises a length dimension and a width dimension, where the length dimension is greater than the width dimension, and the length dimension extends between the first and second projections.

7. A skin treatment apparatus according to any preceding claim wherein the at least first and second projection (14a, 14b) is arranged to be received into a corresponding opening in the housing (4), where each of the projections (14a, 14b) in the rest position extend outwardly from the opening, and in the deflected position the projections are at least partially withdrawn into the opening.

8. A skin treatment apparatus according to any preceding claim wherein the at least first and second projections are rigid.

9. A skin treatment apparatus according to any preceding claim wherein the at least first and second projection are biased to the rest position by a biasing arrangement.

10. A skin treatment apparatus according to any preceding claim wherein at least first and second projection (14a, 14b) are pivotally mounted to the housing (4).

11. A skin treatment apparatus according to claim 10 wherein the skin contact surface comprises a leading edge and a trailing edge, where the leading edge projects further outwardly from the housing in the rest position.

12. A skin treatment apparatus according to any preceding claim wherein the housing has a forward contact surface for contacting a user's skin, where the output window (10) is defined in the forward contact surface, and where the forward contact surface is at least partially convex.

13. A skin treatment apparatus according to any of claims 1-7 and 9-12 when dependent on any of claims 1-7 wherein the at least first and second projection (14a, 14b) are elastically deformable and arranged to conform to the geometry of a user's skin.

14. A skin treatment apparatus according to claim 13 wherein the at least first and second projection (14a, 14b) comprise an elastomeric material.

## Patentansprüche

1. Eine Hautbehandlungsvorrichtung (2) zur Abgabe von Lichtenergieimpulsen an die Haut eines Individuums, wobei die Hautbehandlungsvorrichtung Folgendes beinhaltet:
- ein Gehäuse (4);
- eine Lichtquelle, die in dem Gehäuse untergebracht ist, um Lichtenergieimpulse abzugeben;
- ein Steuersystem zum Steuern der Abgabe der Lichtquelle, um die Lichtenergieimpulse zu liefern;
wobei das Gehäuse (4) ein Ausgangsfenster (10) zur Übertragung der von der Lichtquelle emittierten Lichtenergieimpulse zum Äußeren des Gehäuses auf die Haut des Individuums aufweist, wobei die Hautbehandlungsvorrichtung ferner mindestens einen Vorsprung (14) beinhaltet, der relativ zu dem Ausgangsfenster (10) von dem Gehäuse nach außen vorsteht, und wobei der oder jeder Vorsprung eine Hautkontaktfläche (16) aufweist, wobei der oder jeder Vorsprung mindestens teilweise aus einer Ruheposition in eine abgelenkte Position beweglich ist, sodass der Vorsprung abgelenkt wird, um die Geometrie der Haut des Individuums aufzunehmen, wobei der Vorsprung (16) ferner einen Sensor (18) trägt und wobei das Steuersystem angeordnet ist, um eine oder mehrere Sensorausgaben von dem Sensor zu empfangen und basierend auf der einen oder den mehreren Sensorausgaben den Betrieb der Hautbehandlungsvorrichtung zu steuern, **dadurch gekennzeichnet, dass** der eine oder die mehreren Vorsprünge mindestens einen ersten und einen zweiten Vorsprung (14a, 14b) beinhalten, wobei der erste und der zweite Vorsprung unabhängig voneinander abgelenkt werden.

2. Hautbehandlungsvorrichtung gemäß Anspruch 1, wobei jede der Hautkontaktflächen (16a, 16b) von dem jeweiligen Vorsprung (14a, 14b) nach außen weist.

3. Hautbehandlungsvorrichtung gemäß Anspruch 1, wobei der Sensor (18) von dem Vorsprung (14) umschlossen wird und ferner in einer festen Position relativ zu der Hautkontaktfläche (16) gehalten wird.

4. Hautbehandlungsvorrichtung gemäß einem der vorhergehenden Ansprüche, wobei sich der mindestens erste und zweite Vorsprung (14a, 14b) um die Gesamtheit des Ausgabefensters (10) erstrecken.

5. Hautbehandlungsvorrichtung gemäß einem der vorhergehenden Ansprüche, wobei der eine oder die mehreren Sensoren (18) mindestens erste und zweite Sensoren (18a, 18b) beinhalten, die von dem ersten und zweiten Vorsprung (14a, 14b) auf diametral gegenüberliegenden ersten und zweiten Seiten des Ausgabefensters (10) getragen werden.

6. Hautbehandlungsvorrichtung gemäß einem der vorhergehenden Ansprüche, wobei das Ausgabefenster (10) eine Längenabmessung und eine Breitenabmessung beinhaltet, wobei die Längenabmessung größer als die Breitenabmessung ist und sich die Längenabmessung zwischen dem ersten und zweiten Vorsprung erstreckt.

7. Hautbehandlungsvorrichtung gemäß einem der vorhergehenden Ansprüche, wobei der mindestens erste und zweite Vorsprung (14a, 14b) angeordnet sind, um in einer entsprechenden Öffnung in dem Gehäuse (4) empfangen zu werden, wobei sich jeder der Vorsprünge (14a, 14b) in der Ruheposition von der Öffnung nach außen erstreckt und die Vorsprünge in der abgelenkten Position mindestens teilweise in die Öffnung zurückgezogen sind.

8. Hautbehandlungsvorrichtung gemäß einem der vorhergehenden Ansprüche, wobei der mindestens erste und zweite Vorsprung starr sind.

9. Hautbehandlungsvorrichtung gemäß einem der vorhergehenden Ansprüche, wobei der mindestens erste und zweite Vorsprung durch eine Vorspannanordnung in die Ruheposition vorgespannt sind.

10. Hautbehandlungsvorrichtung gemäß einem der vorhergehenden Ansprüche, wobei der mindestens erste und zweite Vorsprung (14a, 14b) schwenkbar an dem Gehäuse (4) montiert sind.

11. Hautbehandlungsvorrichtung gemäß Anspruch 10, wobei die Hautkontaktfläche eine Vorderkante und eine Hinterkante beinhaltet, wobei die Vorderkante in der Ruheposition weiter nach außen von dem Gehäuse vorsteht.

12. Hautbehandlungsvorrichtung gemäß einem der vorhergehenden Ansprüche, wobei das Gehäuse eine vordere Kontaktfläche zum Kontaktieren der Haut eines Benutzers aufweist, wobei das Ausgabefenster (10) in der vorderen Kontaktfläche definiert ist und wobei die vordere Kontaktfläche mindestens teilweise konvex ist.

13. Hautbehandlungsvorrichtung gemäß einem der Ansprüche 1-7 und 9-12 in Abhängigkeit von einem der Ansprüche 1-7, wobei der mindestens erste und zweite Vorsprung (14a, 14b) elastisch verformbar und angeordnet sind, um sich an die Geometrie der Haut eines Benutzers anzupassen.

14. Hautbehandlungsvorrichtung gemäß Anspruch 13, wobei der mindestens erste und zweite Vorsprung (14a, 14b) ein elastomeres Material beinhalten.

## Revendications

1. Un dispositif de traitement de la peau (2) destiné à l'administration d'impulsions d'énergie lumineuse à la peau d'un sujet, le dispositif de traitement de la peau comprenant :
- un boîtier (4) ;
- une source lumineuse logée à l'intérieur du boîtier destinée à décharger des impulsions d'énergie lumineuse ;
- un système de commande destiné à commander la décharge de la source lumineuse afin d'administrer les impulsions d'énergie lumineuse ;
le boîtier (4) ayant une fenêtre de sortie (10) destinée à la transmission des impulsions d'énergie lumineuse émises par la source lumineuse jusqu'à l'extérieur du boîtier jusque sur la peau du sujet, le dispositif de traitement de la peau comprenant en outre au moins une saillie (14) faisant saillie vers l'extérieur à partir du boîtier par rapport à la fenêtre de sortie (10) et la ou chaque saillie ayant une surface de contact avec la peau (16), dans lequel la ou chaque saillie peut être au moins partiellement déplacée d'une position de repos à une position fléchie de telle sorte que la saillie fléchit pour s'adapter à la géométrie de la peau du sujet, la saillie (16) portant en outre un capteur (18) et où le système de commande est agencé pour recevoir une ou plusieurs sorties de capteur en provenance du capteur et, sur la base des une ou plusieurs sorties de capteur, commander le fonctionnement du dispositif de traitement de la peau, **caractérisé en ce que** les une ou plusieurs saillies comprennent au moins une première et une deuxième saillie (14a, 14b), où les première et deuxième saillies fléchissent indépendamment l'une de l'autre.

2. Un appareil de traitement de la peau selon la revendication 1 dans lequel chacune des surfaces de contact avec la peau (16a, 16b) est tournée vers l'extérieur de la saillie respective (14a, 14b).

3. Un appareil de traitement de la peau selon la revendication 1 dans lequel le capteur (18) est retenu par la saillie (14) et est en outre maintenu dans une position fixe par rapport à la surface de contact avec la peau (16).

4. Un appareil de traitement de la peau selon n'importe quelle revendication précédente dans lequel les au moins première et deuxième saillies (14a, 14b) s'étendent autour de la totalité de la fenêtre de sortie (10).

5. Un appareil de traitement de la peau selon n'importe quelle revendication précédente dans lequel les un ou plusieurs capteurs (18) comprennent au moins des premier et deuxième capteurs (18a, 18b) portés par les première et deuxième saillies (14a, 14b) sur des premier et deuxième côtés diamétralement opposés de la fenêtre de sortie (10).

6. Un appareil de traitement de la peau selon n'importe quelle revendication précédente dans lequel la fenêtre de sortie (10) comprend une dimension de longueur et une dimension de largeur, où la dimension de longueur est supérieure à la dimension de largeur, et la dimension de longueur s'étend entre les première et deuxième saillies.

7. Un appareil de traitement de la peau selon n'importe quelle revendication précédente dans lequel les au moins première et deuxième saillies (14a, 14b) sont agencées pour être reçues dans une ouverture correspondante dans le boîtier (4), où chacune des saillies (14a, 14b) dans la position de repos s'étend vers l'extérieur de l'ouverture, et dans la position fléchie les saillies sont au moins partiellement rétractées dans l'ouverture.

8. Un appareil de traitement de la peau selon n'importe quelle revendication précédente dans lequel les au moins première et deuxième saillies sont rigides.

9. Un appareil de traitement de la peau selon n'importe quelle revendication précédente dans lequel les au moins première et deuxième saillies sont décalées vers la position de repos par un agencement de décalage.

10. Un appareil de traitement de la peau selon n'importe quelle revendication précédente dans lequel les au moins première et deuxième saillies (14a, 14b) sont montées de manière pivotante sur le boîtier (4).

11. Un appareil de traitement de la peau selon la revendication 10 dans lequel la surface de contact avec la peau comprend un bord d'attaque et un bord de fuite, où le bord d'attaque fait saillie davantage vers l'extérieur du boîtier dans la position de repos.

12. Un appareil de traitement de la peau selon n'importe quelle revendication précédente dans lequel le boîtier a une surface de contact avant destinée à entrer en contact avec la peau d'un utilisateur, où la fenêtre de sortie (10) est définie dans la surface de contact avant, et où la surface de contact avant est au moins partiellement convexe.

13. Un appareil de traitement de la peau selon n'importe lesquelles des revendications 1 à 7 et 9 à 12 lorsqu'elles dépendent de n'importe lesquelles des revendications 1 à 7 dans lequel les au moins première et deuxième saillies (14a, 14b) sont élastiquement déformables et agencées pour épouser la géométrie de la peau d'un utilisateur.

14. Un appareil de traitement de la peau selon la revendication 13 dans lequel les au moins première et deuxième saillies (14a, 14b) comprennent un matériau élastomère.
